# EUROPEAN PATENT SPECIFICATION

(11) **EP 4 259 276 B1**
(45) Date of publication and mention of the grant of the patent: **29.01.2025**
(21) Application number: 21820286.9
(22) Date of filing: 09.12.2021
(51) Int. Cl.: A61N 2/02, H01F 27/02, H01F 27/10, H01F 27/28, H01F 27/30

(54) **APPLICATOR FOR MAGNETIC THERAPY**
APPLIKATOR FÜR DIE MAGNETISCHE THERAPIE
APPLICATEUR POUR THÉRAPIE MAGNÉTIQUE

(30) Priority: 11.12.2020 IT 202000030524
(43) Date of publication of application: 18.10.2023
(73) Proprietor: EL.EN. S.p.A., 50041 Calenzano (FI) (IT)
(72) Inventor: TAGLIAFERRI, Marco, 50041 Calenzano (FI) (IT); MASOTTI, Leonardo, (deceased) (IT); BENI, Samuele, 50041 Calenzano (FI) (IT); BUSCEMI, Calogero, 50041 Calenzano (FI) (IT)
(74) Representative: Mannucci, Michele
(86) International application number: PCT/EP2021/085004
(87) International publication number: WO 2022/122923

(56) References cited:
- CN-A- 109 276 812
- KR-A- 20030 065 126
- KR-B1- 102 046 924
- US-A1- 2006 004 244
- US-B1- 10 569 095

## Description

### TECHNICAL FIELD

The present invention relates to medical equipment for magnetic therapy treatments. In more detail, the invention relates to improvements to magnetic therapy applicators.

### BACKGROUND TO THE INVENTION

Devices and methods for generating magnetic pulses have been used for a long time for medical and aesthetic treatments. To this end, applicators are used, in which electrically conductive coils are provided. Electric current, variable over time, flows in the coils and generates variable magnetic fields. By putting the applicators onto the patient's body or close thereto, the variable magnetic fields enter the patient's body and induce in it electric currents having an effect similar to that of electro-therapy treatments.

Magnetic field treatments may have both aesthetic and medical purposes, for example stimulating muscle in rehabilitation, or the like. Magnetic field treatments may be effectively used in medical applications such as fat reduction, thus lessening the risks connected to pre-obesity and obesity, with particular reference to the abdominal region. With reference to the medical sector again, magnetic field treatments may be effectively used for strengthening the pelvic floor muscle in in the treatment of urinary incontinence. Magnetic therapy may be also used in the aesthetic sector for body shaping.

The electric currents induced in biological tissues by the magnetic fields cause, among other things, neural excitation and stimulation of muscle contractions. The intensity thereof depends on the magnetic flow density and the law of change over time of the magnetic flow.

The treatment aims at achieving a muscle stimulation of such intensity, frequency and repetitions to cause a muscle supramaximal contraction, i.e. level and duration of the contraction significantly higher than the physiological ones (maximal voluntary contractions). In this way, muscle stresses and physiological workload increase, requiring the muscle to adapt to these extreme conditions by deeply sculpting its inner structure through hypertrophy and hyperplasia processes resulting in an increase of muscle volume and tone.

Magnetic therapy applicators (coils) shall be therefore able to generate a pulsed magnetic field, whose single pulse shall have such a duration to induce neuromuscular stimulation. The pulse duration is typically comprised between 200 µs and 300 µs. Moreover, the magnetic therapy applicator (coil) shall be able to generate the pulses with such an intensity as to penetrate deeply, reaching the muscle or tissue region to be treated, onto which neuromuscular stimulation shall be induced.

Therefore, as the magnetic field intensity and spatial distribution also depend on the coil geometry, typically different coils are manufactured according to the type of region/tissue to be treated and to the depth to be achieved. In addition to achieve the neuro-stimulation threshold in the area to be treated and at the required depth, the magnetic therapy applicator shall be also able to support the generation of magnetic field pulse sequences of variable frequency, adapted to induce muscle supra-maximal contractions or the contractions required by the specific treatment. These contractions, and therefore the pulses generating them, shall follow defined patterns, which typically involve also changes in the magnetic field intensity, because, usually, during a same treatment muscle contraction and relaxation phases shall alternate with one another. Typically, the maximal frequency used is approximately 150 Hz. The overall duration of the treatment, intended as sequences of patterns of magnetic field pulses of variable frequency and intensity, typically varies from 15 minutes to 45 minutes, depending on the patient.

Magnetic therapy applicators comprise a housing with an outer wall, which defines a contact surface, i.e. an application surface to be applied to the patient. At least one electrically conductive coil is arranged in the housing for generating a magnetic field. A refrigerating device shall be associated with the applicator, in order to avoid overheating of the coil and above all thermal injuries to the patient. Some applicators are therefore equipped with a fan generating a refrigerating airflow that removes heat from the applicator whilst current passes in the coil.

In order to have quick results with a few treatments, the treatments shall advantageously provide for prolonged steps of supramaximal contraction and high duty cycles. This can be achieved only by applying sequences of magnetic field pulses of high intensity and frequency. Due to these prolonged phases, the coil is passed by high intensity currents for most treatment time, with consequent heat generation due both to the Joule effect and the proximity effect of the magnetic field generated by the same coil.

Therefore, a hindrance to the prolonged application of high currents is the risk of burns to the patient due to the heat generated by the coil.

US-B-10,569,095 discloses a magnetic therapy applicator comprising a housing with an outer wall, which defines an application surface to be applied to a patient, and in which at least one electrically conductive coil is housed for generating a magnetic field. The coil is so installed in the housing that a gap is formed between the coil face facing the patient and the application surface to be applied to the patient, and air forced circulation for heat removal is generated in this gap by a fan. This refrigerating system is poorly efficient.

KR20030065126, KR102046924, CN109276812 and US2006/004244 disclose further application devices. These devices are poorly efficient too as regards heat removal during treatment.

There is therefore a need to improve magnetic therapy applicators in order to allow efficient treatments in short time without the risk of thermal injuries to the patient or thermal damages to the devices contained in the applicator.

### SUMMARY

The present invention is defined by the appended claims.

The present disclosure relates to an applicator for magnetic therapy comprising a housing with an outer wall, which defines an application surface to be applied to a patient, and in which at least one electrically conductive coil is housed for generating a magnetic field. In order to improve its features, the applicator is also provided with an outer shell forming the outer wall and constituting the application surface, and an inner shell forming a seat for housing the coil. The inner shell comprises a bottom wall facing the inner surface of the outer shell and, more precisely, facing the outer wall defining the application surface of the outer shell.

A gap is advantageously formed between the bottom wall of the inner shell and the outer wall of the outer shell for thermally insulating the coil and the application surface from each other.

The use of an outer shell and an inner shell makes the production of the applicator simpler, as it is possible to provide, in the inner shell, the magnetic field generating components, the temperature controllers, for instance an NTC resistance or other temperature sensor, as well as, if necessary, a refrigerating device. These components may be resin-encapsulated to form a single block to be inserted in the outer shell. Resin-encapsulation gives the coil housing the necessary mechanical stability, also ensuring adequate electrical insulation and good thermal conductivity of heat generated in the coil. The outer shell may be closed by a closing shell.

Moreover, the gap between the outer wall of the outer shell forming the application surface, and the bottom wall of the inner shell constitutes an efficient thermal barrier for reducing heat transfer from the coil to the application surface and, therefore, to the patient. In this way, very intense magnetic fields can be generated, thus making the treatment effective and quick, avoiding thermal injuries to the patient and making the treatment more pleasant.

The applicator further comprises a refrigerating device.

Specifically, the refrigerating device comprises a fluid circulating system with at least one refrigerating duct adapted to circulate a refrigerating fluid, for example water. However, the refrigerating fluid is preferably an electrically low conductive fluid, in order to avoid generation of parasite currents in the coils formed by the refrigerating duct, which parasite currents are able to generate magnetic fields opposing the variable magnetic field generated by the coil.

Typically, high dielectric strength oils (generally utilized for cooling of transformers) are used, or alternatively demineralized water, wherein, during operation, conductibility control systems ensure low conductibility values.

The refrigerating duct is suitably arranged between the coil and the application surface, for more effective refrigerating of the applicator area touching the patient.

In particular, the refrigerating duct may be arranged between the coil and an outer surface of the bottom wall of the inner shell, preferably in a seat or groove formed in the surface of the bottom wall facing the coil.

In particularly advantageous embodiments, the refrigerating duct is spiral shaped, lying on a plane parallel to the bottom surface of the inner shell and housed in a spiral groove formed in the thickness of the bottom wall and open towards the coil.

In alternative embodiments, ducts or channels may be provided in the thickness of the bottom wall of the inner shell, for example by additive manufacturing.

In the thermal insulation gap formed between the outer wall of the outer shell and the bottom wall of the inner shell, spacing elements are provided, which define the height of the gap, i.e. the distance between the surface of the outer wall facing the inner shell and the surface of the bottom wall of the inner shell facing the outer wall of the outer shell.

In order to reduce the contact between the outer shell and the inner shell at the gap, the spacing elements can be approximately dot-shaped, i.e., they can have the shape of prisms, cones or cylinders with a small cross section. This allows reducing conductive heat transfer between the inner shell and the outer shell by realizing a thermal break between the inner shell and the outer shell.

If the inner shell comprises a groove for housing the refrigerating duct, the spacing elements are preferably arranged at the groove, where the temperature is lowest due to the refrigerating fluid. This further reduces heat transfer.

Further features and embodiments of the applicator will be described below with reference to the attached drawing.

### BRIEF DESCRIPTION OF THE DRAWING

The invention will be better understood by following the description below and the attached drawing, showing a non-limiting embodiment of the invention. More specifically, in the drawing:
Fig. 1 is an axonometric view of an applicator;

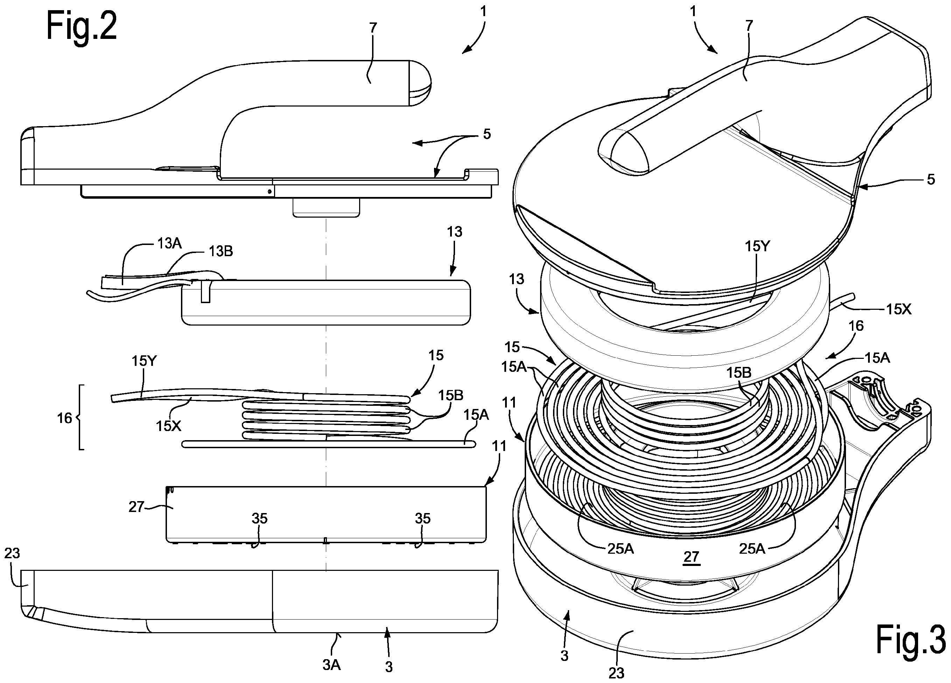

Fig. 2 is an exploded side view of the applicator of Fig. 1;
Fig. 3 is an exploded axonometric view;

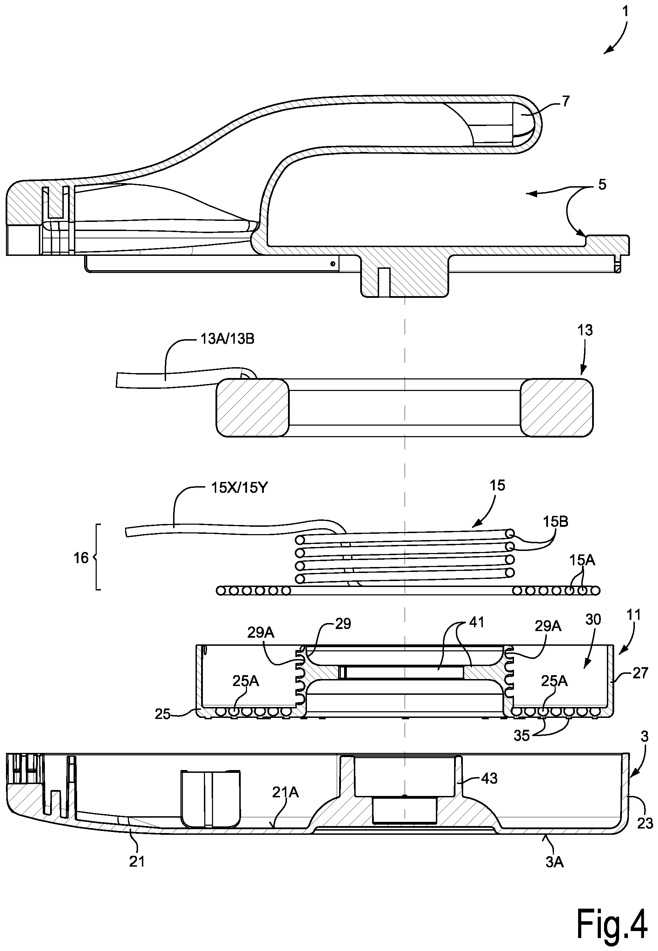

Fig. 4 is an exploded sectional view;
Fig. 5 is a cross-section of the assembled applicator according to a plane orthogonal to the application surface to be applied to the patient;
Fig. 6 is an enlargement of detail VI of Fig. 5;
Fig. 7 is a cross-section according to VII-VII of Fig. 5; and
Fig. 8 is an axonometric view of the applicator without the top covering.

### DETAILED DESCRIPTION

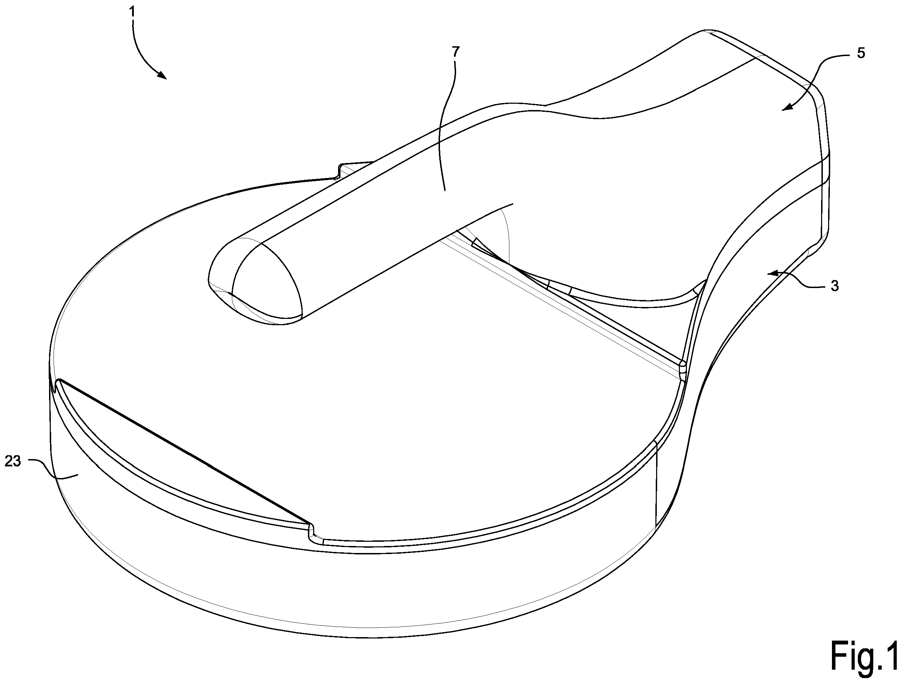

Fig. 1 is an outer axonometric view of an applicator 1. In the illustrated embodiment, the applicator 1 comprises an outer shell 3, which defines the application surface to be applied to the patient, and a covering shell 5. The outer shell 3 and the covering shell 5 form a closed housing or casing, in which one or more electromagnetic coils are provided, i.e. coils adapted to generate a magnetic field when current flows therein, and a refrigerating device. The surface of the applicator to be applied to the patient is indicated with reference number 3A and will be briefly referred to below as application surface.

In the embodiment illustrated just by way of non-limiting example, the applicator 1 comprises a handle 7 that, in this case, is an integral part of the covering shell 5.

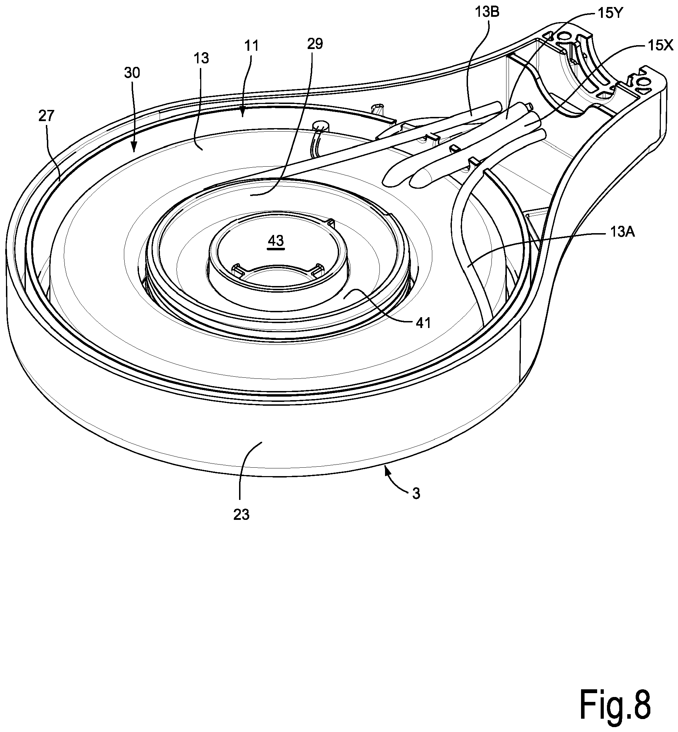

An inner shell 11 is provided inside the volume formed between the outer shell 3 and the covering shell 5; the inner shell 11 forms a support for receiving a coil 13, made of electrically conductive material, and a refrigerating device 16 , which in the illustrated embodiment consists of a refrigerating circuit configured to circulate a refrigerating liquid. In the illustrated embodiment, the refrigerating circuit comprises only one refrigerating duct 15 forming multiple turns, as detailed below. The reference numbers 13A, 13B (see in particular Fig. 8) indicate the power supply terminals of the coil 13, whilst the numbers 15X, 15Y indicate the inlet and outlet of the refrigerating duct 15.

In the illustrated embodiment, the refrigerating duct 15 is constituted by a tube manufactured separately from the inner shell 11 and inserted in a seat, for example a groove, provided in the bottom wall of the inner shell 11. However, different embodiments are also possible. The bottom wall may be for example comprised of two mutually coupled components, between which the refrigerating duct 15 is formed. In further embodiments, the refrigerating duct may be formed, by additive manufacturing, in the bottom wall of the inner shell 11.

The outer shell 3 comprises an outer wall 21, which defines the application surface 3A. A side wall 23 extends from the outer wall 21, approximately orthogonally to the application surface 3A; the side wall 23 surrounds the inner shell 11 and is coupled to the covering shell 5.

The inner shell 11 has an overall approximately cylindrical shape and comprises an annular bottom wall 25, from the outer edge whereof an approximately cylindrical first perimeter wall 27 extends, approximately orthogonally to the bottom wall 25. An approximately cylindrical second perimeter wall 29, substantially coaxially with the first perimeter wall 27, extends from an inner edge of the bottom wall 25.

The walls 25, 27, 29 define an annular seat 30, where the coil 13 and the refrigerating device 16 are housed as described below. The coil 13 and the refrigerating device 16, i.e. the refrigerating duct 15, are advantageously resin-encapsulated in the annular seat 30. Resin-encapsulation is not shown in the attached drawing for greater clarity.

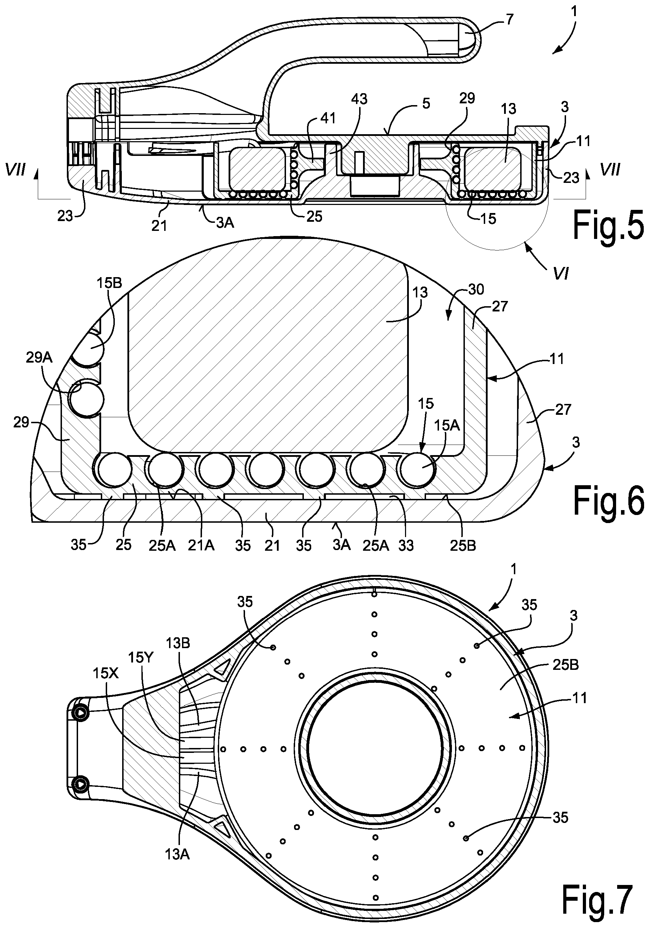

As mentioned above, in the illustrated embodiment the refrigerating circuit constituting the refrigerating device 16 comprises a single refrigerating duct 15, which forms a spiral, lying on a plane parallel to the bottom wall 25, and is partially embedded in the thickness of the bottom wall 25. In the illustrated embodiment, as shown in particular in the detail of Fig. 6, a seat is provided in the inner surface of the bottom wall 25 for housing part of the refrigerating duct 15. The seat is formed by a spiral-shaped groove 25A, where the turns 15A of the refrigerating duct 15 are housed. The thickness of the bottom wall 25 is lower along the spiral-shaped groove 25A than in the area between adjacent turns.

Configuring the refrigerating circuit with only one spiral-shaped refrigerating duct is particularly advantageous and makes the production easy. However, further embodiments are also possible, for example with a greater number of refrigerating ducts, or with a matrix of refrigerating channels provided in the thickness of the bottom wall 25 and connected to an inlet and an outlet of a refrigerating fluid. The channels may be produced, for example, by additive manufacturing.

In the illustrated embodiment, the refrigerating duct 15 forms a further plurality of helical turns, which coil outside the second perimeter wall 29. The helical coils, indicated with reference number 15B, are housed in a helical groove 29A provided in the thickness of the second perimeter wall 29.

In assembled condition, the bottom wall 25 and the outer wall 21 are approximately parallel to each other and form a gap 33 (see in particular Fig. 6) for thermally insulating the coil 13 and the outer wall 21 from each other. More specifically, the gap is defined between a surface 25B of the bottom wall 25 facing the outer wall 21 and a surface 21A of the outer wall 21 facing the inner shell 11.

The thickness, i.e. the height of the gap 33, is extremely small, for example in the order of 0.5-5 mm, preferably about 0.5-1 mm. Spacing elements 35 are provided to keep the distance between the surface 25B and the surface 21A (this distance being the thickness or height of the gap 33). The spacing elements 35 are advantageously arranged in radial rows, as shown in particular in Fig. 7.

In order to reduce the contact between the outer shell 3 and the inner shell 11 at the gap 33, the spacing elements are, for instance, dot-shaped, i.e., they are shaped like small (for example cylindrical) bodies that extend orthogonally to the surfaces delimiting the gap 33 and have very small cross-section, for example a maximum transverse dimension equal to, or lower than, 2 mm. In case the spacing elements 35 are cylindrical, the maximum transverse dimension is given by the base diameter thereof.

Furthermore, in the illustrated embodiment the spacing elements 35 are arranged in correspondence of the turns of the groove 25A, as clearly shown in Fig. 6A, for purposes that will be explained below. In case the refrigerating duct 15 is directly formed, for example by additive manufacturing, in the thickness of the bottom wall 25 of the inner shell 11, the spacing elements 35 may be provided again in correspondence of grooves constituting the refrigerating duct.

The spacing elements 35 may be made in a single piece with the outer shell 3 or the inner shell 11. The spacing elements 35 are preferably integral with the inner shell 11. In this way, by producing the inner shell 11 for example by molding or by additive manufacturing, it is possible to position the spacing elements 35 precisely in correspondence of the areas where the bottom wall 25 is less thick, i.e. in correspondence of the grooves 25A.

In the illustrated embodiment, inside the second perimeter wall 29 a collar 41 is provided, coaxial to the walls 29 and 27. The collar 41 constitutes a centering member between the inner shell 11 and the outer shell 3. The collar 41 co-acts with a shank 43 integral with the outer shell 3. In assembled condition (Fig. 5) the shank 43 is inserted inside the collar 41.

With the arrangement described above, thermal insulation is provided between the inner shell 11, which houses the coil 13 generating heat, and the application surface 3A. Thermal insulation is provided by the gap 33 filled with air, which is an effective thermal insulator. Thanks to the use of small spacing elements 35, the conductive heat transfer from the coil 13 to the application surface 3A is significantly reduced. Typically, the overall area of the contact surfaces between the outer shell and the inner shell in correspondence of the gap 33 is equal to, or lower than 1%, and preferably equal to, or lower than 0.8% of that of the cross-section (according to plane VII-VII of Fig. 5) of the gap 33.

The heat transfer between the inner shell 11 and the outer shell 3 is further reduced thanks to the fact that the spacing elements 35 are provided in correspondence of the groove 25A, i.e. where the refrigerating fluid circulating in the duct 15 flows and therefore, the temperature of the material forming the bottom wall 25 is minimal.

The above description illustrates one embodiment of an applicator according to the invention. It will be clearly apparent to those skilled in the art that many variants are possible, without departing from the scope of the invention as defined in the attached claims. The present invention is defined by the appended claims.

## Claims

1. An applicator (1) for magnetic therapy comprising a housing with an outer wall (21), which defines an application surface (3A) to be applied to a patient, and in which at least one electrically conductive coil (13) is housed for generating a magnetic field; wherein:
the housing comprises: an outer shell (3) forming the outer wall (21); and an inner shell (11) forming a seat (30) for housing the coil (13), wherein the inner shell (11) comprises a bottom wall (25) facing the outer wall (21) formed by the outer shell (3);
in the housing, a refrigerating device (16) is provided, comprising at least one refrigerating duct (15) adapted to circulate a refrigerating fluid and arranged between the coil (13) and the application surface (3A); wherein the at least one refrigerating duct (15) is arranged between the coil (13) and a surface (25B) of the bottom wall (25) of the inner shell (11) facing the outer wall (21) of the outer shell (3);
a gap (33) is formed between the bottom wall (25) of the inner shell (11) and the outer wall (21) of the outer shell (3) for thermally insulating the coil (13) and the application surface (3A) from each other; and
spacing elements (35) are arranged in the thermal insulation gap (33) between the outer wall (21) of the outer shell (3) and the bottom wall (25) of the inner shell (11).

2. The applicator of claim 1, wherein the at least one refrigerating duct (15) in arranged in the inner shell (11) between the coil and an inner surface of the bottom wall (25) of the inner shell (11).

3. The applicator (1) of claim 1 or 2, wherein a height of the gap thermal insulation (33) comprised between 0.5 and 5 mm, preferably between 0.5 and 1 mm.

4. The applicator (1) of one or more of the previous claims, wherein the bottom wall (25) of the inner shell (11) has at least one groove (25A), which forms, or in which is inserted, the refrigerating duct (15); wherein the bottom wall (25) has reduced thickness in correspondence of the at least one groove (25A); and wherein the groove (25A) is preferably open on the face of the bottom wall (25) facing the coil (13).

5. The applicator (1) of claim 4, wherein the groove (25A) is spiral-shaped.

6. The applicator (1) of one or more of the previous claims, wherein the spacing elements (35) are integral with the outer wall (21) of the outer shell (3) or with the bottom wall (25) of the inner shell (11) and define a mutual contact surface between the bottom wall (25) and the outer wall (21) that is lower than 1% of the surface of the cross section of the thermal insulation gap (33).

7. The applicator (1) of one or more of the previous claims, wherein the spacing elements (35) comprise dot-shaped elements.

8. The applicator (1) of one or more of the previous claims, wherein the spacing elements (35) are arranged according to alignments that are radial with respect to the axis of the coil (13).

9. The applicator (1) of one or more of the previous claims when depending on at least claim 3, wherein the spacing elements (35) are arranged in correspondence of the at least one groove (25A).

10. The applicator (1) of one or more of the previous claim, wherein the inner shell (11) has: a first perimeter wall (27) extending approximately orthogonal to the bottom wall (25) along an outer perimeter edge of the bottom wall (25), a second perimeter wall (29) approximately coaxial with the first perimeter wall (27) and extending approximately orthogonal to the bottom wall (25) along an inner perimeter edge of the bottom wall (25); the first perimeter wall (27), the second perimeter wall (29) and the bottom wall (25) defining an annular seat (30) for the coil (13).

11. The applicator (1) of claim 10, wherein the second perimeter wall (29) has at least a further groove (29A), which forms the refrigerating duct (15), or in which the refrigerating duct (15) is inserted; in particular the further groove (29A) extending helically along the second perimeter wall (29)

12. The applicator (1) of claim 10 or 11, wherein the outer shell (3) comprises a shank (43), which is approximately orthogonal to the outer wall (21) and is inserted coaxially with respect to the second perimeter wall (29), the shank co-acting with a centering member (41) associated with the second perimeter wall (29).

13. The applicator (1) of one or more of the previous claims, wherein the outer shell (3) comprises a side wall (23), which is approximately orthogonal to the outer wall (21) and perimetrically surrounds the inner shell (11).

14. The applicator (1) of one or more of the previous claims, wherein at least one of said coil (13) and said at least one refrigerating duct (15) is resin-encapsulated in the inner shell (11).

15. The applicator (1) of one or more of the previous claims, comprising a covering shell (5) adapted to form, with the outer shell (3), a closing casing where the inner shell (11), the refrigerating device (16) and the coil (13) are arranged.

## Patentansprüche

1. Applikator (1) für Magnettherapie mit einem Gehäuse mit einer äußeren Wand (21), die eine Anlegefläche (3A) definiert, die an einen Patienten angelegt wird, und in der mindestens eine elektrisch leitfähige Spule (13) aufgenommen ist, um ein Magnetfeld zu erzeugen, wobei:
das Gehäuse aufweist: eine äußere Hülle (3), die die äußere Wand (21) bildet, und eine innere Hülle (11), die einen Sitz (30) zur Aufnahme der Spule (13) bildet, wobei die innere Hülle (11) eine untere Wand (25) aufweist, die zu der äußeren Wand (21), die durch die äußere Hülle (3) gebildet ist, gerichtet ist,
wobei in dem Gehäuse eine Kühlvorrichtung (16) vorgesehen ist, die mindestens eine Kühlleitung (15 aufweist, die ausgebildet ist, um ein Kühlfluid zu zirkulieren, und die zwischen der Spule (13) und der Anlegefläche (3A) angeordnet ist, wobei die mindestens eine Kühlleitung (15) zwischen der Spule (13) und einer Fläche (25B) der unteren Wand (25) der inneren Hülle (11), die zu der äußeren Wand (21) der äußeren Hülle (3) gerichtet ist, angeordnet ist,
ein Spalt (33) zwischen der unteren Wand (25) der inneren Hülle (11) und der äußeren Wand (21) der äußeren Hülle (3) zum thermischen Isolieren der Spule (13) und der Anlegefläche (3A) von einander ausgebildet ist, und
Abstandselemente (35) in dem thermisch isolierenden Spalt (33) zwischen der äußeren Wand (21) der äußeren Hülle (3) und der unteren Wand (25) der inneren Hülle (11) angeordnet sind.

2. Applikator nach Anspruch 1, wobei die mindestens eine Kühlleitung (15) in der inneren Hülle (11) zwischen der Spule und einer inneren Fläche der unteren Wand (25) der inneren Hülle (11) angeordnet ist.

3. Applikator (1) nach Anspruch 1 oder 2, wobei eine Höhe des Spaltes der thermischen Isolation (33) zwischen 0,5 und 5 mm, vorzugsweise zwischen 0,5 und 1 mm beträgt.

4. Applikator nach einem oder mehreren der vorstehenden Ansprüche, wobei die untere Wand (25) der inneren Hülle (11) mindestens eine Nut (25A) aufweist, die die Kühlleitung (15) bildet oder in die sie eingebracht ist, wobei die untere Wand (25) in Übereinstimmung mit der mindestens einen Nut (25A) eine reduzierte Dicke aufweist und wobei die Nut (25A) vorzugsweise auf der Fläche der unteren Wand (25), die zu der Spule (13) gerichtet ist, offen ist.

5. Applikator (1) nach Anspruch 4, wobei die Nut (25A) spiralförmig ist.

6. Applikator (1) nach einem oder mehreren der vorstehenden Ansprüche, wobei die Abstandselemente (35) einstückig mit der äußeren Wand (21) der äußeren Hülle (3) oder mit der unteren Wand (25) der inneren Hülle (11) sind und eine gegenseitige Kontaktfläche zwischen der unteren Wand (25) und der äußeren Wand (21) bilden, die weniger als 1 % der Fläche des Querschnitts des thermisch isolierenden Spaltes (33) beträgt.

7. Applikator (1) nach einem oder mehreren der vorstehenden Ansprüche, wobei die Abstandselemente (35) punktförmige Elemente aufweisen.

8. Applikator (1) nach einem oder mehreren der vorstehenden Ansprüche, wobei die Abstandselemente (35) entsprechend Ausrichtungen angeordnet sind, die radial mit Bezug auf die Achse der Spule (13) sind.

9. Applikator (1) nach einem oder mehreren der vorstehenden Ansprüche, wenn sie mindestens von Anspruch 3 abhängen, wobei die Abstandselemente (35) in Entsprechung mit der mindestens einen Nut (25A) angeordnet sind.

10. Applikator (1) nach einem oder mehreren der vorstehenden Ansprüche, wobei die innere Hülle (11) aufweist: eine erste Umfangswand (27), die sich etwa orthogonal zu der unteren Wand (25) entlang einer äußeren Umfangskante der unteren Wand (25) erstreckt, eine zweite Umfangswand (29), die etwa koaxial mit der ersten Umfangswand (27) ist und sich etwa orthogonal zu der unteren Wand (25) entlang einer inneren Umfangskante der unteren Wand (25) erstreckt, wobei die erste Umfangswand (27), die zweite Umfangswand (29) und die untere Wand (25) einen ringförmigen Sitz (30) für die Spule (13) bilden.

11. Applikator (1) nach Anspruch 10, wobei die zweite Umfangswand (29) mindestens eine weitere Nut (29A) aufweist, die die Kühlleitung (15) bildet oder in die die Kühlleitung (15) eingefügt ist, wobei insbesondere die weitere Nut (29A) sich wendelförmig entlang der zweiten Umfangswand (29) erstreckt.

12. Applikator (1) nach Anspruch 10 oder 11, wobei die äußere Hülle (3) einen Schaft (43) aufweist, der etwa orthogonal zu der äußeren Wand (21) ist und koaxial mit Bezug auf die zweite Umfangswand (29) eingefügt ist, wobei der Schaft mit einem Zentrierelement (41) zusammenwirkt, das der zweiten Umfangswand (29) zugeordnet ist.

13. Applikator (1) nach einem oder mehreren der vorstehenden Ansprüche, wobei die äußere Hülle (3) eine Seitenwand (23) aufweist, die etwa orthogonal zu der äußeren Wand (21) ist und die innere Hülle (11) umfangsmäßig umgibt.

14. Applikator (1) nach einem oder mehreren der vorstehenden Ansprüche, wobei zumindest eines von der Spule (13) und der mindestens einen Kühlleitung (15) durch Harz in der inneren Hülle (11) eingekapselt ist.

15. Applikator (1) nach einem oder mehreren der vorstehenden Ansprüche mit einer abdeckenden Hülle (5), die ausgebildet ist, um mit der äußeren Hülle (3) ein Verschlussgehäuse zu bilden, wo die innere Hülle (11), die Kühlvorrichtung (16) und die Spule (13) angeordnet sind.

## Revendications

1. Un applicateur (1) pour une thérapie magnétique, comprenant un logement avec une paroi externe (21) qui forme une surface d'application (3A) à appliquer sur un patient, et dans lequel au moins une bobine électriquement conductrice (13) est logée pour générer un champ magnétique ; dans lequel
le logement comprend : une coque externe (3) formant la paroi externe (21) ; et une coque interne (11) formant un logement (30) pour loger la bobine (13), la coque interne (11) comprenant une paroi inférieure (25) en face de la paroi externe (21) formée par la coque externe (3) ;
dans le logement, un dispositif de réfrigération (16) est prévu, comprenant au moins un conduit de réfrigération (15) apte à faire circuler une fluide réfrigérant et agencé entre la bobine (13) et la surface d'application (3A) ; le ou les conduits de réfrigération (15) étant agencé(s) entre la bobine (13) et une surface (25B) de la paroi inférieure (25) de la coque interne (11) en face de la paroi externe (21) de la coque externe (3) ;
un espace (33) est formé entre la paroi inférieure (25) de la coque interne (11) et la paroi externe (21) de la coque externe (3) pour isoler thermiquement la bobine (13) et la surface d'application l'une de l'autre ; et
des éléments d'espacement (35) sont agencés dans l'espace d'isolation thermique (33) entre la paroi externe (21) de la coque externe (3) et la paroi inférieure (25) de la coque interne (11).

2. L'applicateur selon la revendication 1, dans lequel le ou les conduit(s) de réfrigération (15) est/sont agencé(s) dans la coque interne (11) entre la bobine et une surface interne de la paroi inférieure (25) de la coque interne (11).

3. L'applicateur (1) selon la revendication 1 ou 2, dans lequel la hauteur de l'espace d'isolation thermique (33) comprend entre 0,5 et 5 mm, de préférence entre 0,5 et 1 mm.

4. L'applicateur (1) selon l'une ou plusieurs des revendications précédentes, dans lequel la paroi inférieure (25) de la coque interne (11) comporte au moins une rainure (25A), qui forme ou dans laquelle est inséré le conduit de réfrigération (15) ; la paroi inférieure (25) comportant une épaisseur réduite en correspondance avec la ou les rainure(s) (25A) ; et la rainure (25A) étant de préférence ouverte sur la face de la paroi inférieure (25) faisant face à la bobine (13).

5. L'applicateur (1) selon la revendication 4, dans lequel la rainure (25A) est en forme de spirale.

6. L'applicateur (1) selon l'une ou plusieurs des revendications précédentes, dans lequel les éléments d'espacement (25) sont solidaires de la paroi externe (21) de la coque externe (3) ou de la paroi inférieure (25) de la coque interne (11) et forment un surface de contact mutuel entre la paroi inférieure (25) et la paroi externe (21) qui est inférieure à 1% de la surface de la section transversale de l'espace d'isolation thermique (33).

7. L'applicateur (1) selon l'une ou plusieurs des revendications précédentes, dans lequel les éléments d'espacement (35) comprennent des éléments en forme de points.

8. L'applicateur (1) selon l'une ou plusieurs des revendications précédentes, dans lequel les éléments d'espacement (35) sont agencés suivant des alignements qui sont radiaux par rapport à l'axe de la bobine (13).

9. L'applicateur (1) selon l'une ou plusieurs des revendications précédentes, lorsqu'elles dépendent de la revendication 3 au moins, dans lequel les éléments d'espacement (35) sont agencés en correspondance avec la ou les rainure(s) (25A).

10. L'applicateur (1) selon l'une ou plusieurs des revendications précédentes, dans lequel la coque interne (11) a : une première paroi périmétrique (27) s'étendant approximativement perpendiculairement à la paroi inférieure (25) le long d'un bord périmétrique externe de la paroi inférieure (25), une deuxième paroi périmétrique (29) approximativement coaxiale avec a première paroi périmétrique (27) et s'étendant approximativement perpendiculairement à la paroi inférieure (25) suivant un bord périmétrique interne de la paroi inférieure (25) ; la première paroi périmétrique (27), la deuxième paroi périmétrique (29) et la paroi inférieure (25) formant un logement annulaire (30) pour la bobine (13).

11. L'applicateur (1) selon la revendication 10, dans lequel la deuxième paroi périmétrique (29) comporte au moins une rainure supplémentaire (29A), qui forme le conduit de réfrigération (15) ou dans laquelle le conduit de réfrigération (15) est inséré ; la rainure supplémentaire (29A) s'étendant en particulier hélicoïdalement le long de la deuxième paroi périmétrique (29).

12. L'applicateur (1) selon la revendication 10 ou 11, dans lequel la coque externe (3) comprend une tige (43) qui est approximativement perpendiculaire à la paroi externe (21) et introduite de manière coaxiale par rapport à la deuxième paroi périmétrique (29), la tige coopérant avec un organe de centrage (41) associé à la deuxième paroi périmétrique (29).

13. L'applicateur (1) selon l'une ou plusieurs des revendications précédentes, dans lequel la coque externe (3) comprend une paroi latérale (23), qui est approximativement perpendiculaire à la paroi externe (21) et entoure de manière périmétrique la coque interne (11).

14. L'applicateur (1) selon l'une ou plusieurs des revendications précédentes, dans lequel au moins l'un de ladite bobine (13) et dudit ou desdits conduit(s) de réfrigération (15) est encapsulé par de la résine dans la coque interne (11).

15. L'applicateur (1) selon l'une ou plusieurs des revendications précédentes, comprenant une coque de couverture (5) apte à former avec la coque externe (3), un boîtier fermant où la coque interne (11), le dispositif de réfrigération (16) et la bobine (13) sont agencés.
